# EUROPEAN PATENT SPECIFICATION

(11) **EP 3 558 267 B1**
(45) Date of publication and mention of the grant of the patent: **26.04.2023**
(21) Application number: 16819066.8
(22) Date of filing: 20.12.2016
(51) Int. Cl.: A61K 9/08, A61K 47/10, A61K 47/18, A61K 47/32, A61K 47/36, A61K 31/465

(54) **A MUCOADHESIVE OROMUCOSAL FORMULATION COMPRISING A NICOTINE COMPLEX**
MUKOADHÄSIVE OROMUKOSALE FORMULIERUNG MIT EINEM NIKOTINKOMPLEX
PRÉPARATION OROMUQUEUSE MUCOADHÉSIVE COMPRENANT UN COMPLEXE DE NICOTINE

(43) Date of publication of application: 30.10.2019
(73) Proprietor: Fertin Pharma A/S, 7100 Vejle (DK)
(72) Inventor: NIELSEN, Kent Albin, 7330 Brande (DK)
(74) Representative: Patentgruppen A/S
(86) International application number: PCT/EP2016/081878
(87) International publication number: WO 2018/113916

(56) References cited:
- WO-A1-94/27576
- WO-A1-2008/037470
- WO-A1-2012/083947
- WO-A1-2013/143891
- WO-A2-2009/074552
- US-A1- 2007 269 386
- US-B1- 6 238 689

## Description

### Field of the invention

The present invention relates to a liquid mouth spray device containing a liquid mucoadhesive oromucosal formulation comprising a nicotine complex according to the claims. The invention further relates to methods producing a liquid mouth spray devices and liquid or gel mucoadhesive oromucosal formulation according to the claims.

### Background

Nicotine (NCT) is the main alkaloid found in tobacco and responsible for an addictive potential.

Quitting smoking can significantly improve quality of life, and therefore nicotine replacement therapy (NRT) is of great importance. NRT is available in different dosage forms such as gums, nasal and mouth sprays, sublingual tablets, lozenges, buccal films and transdermal patches. Mouth sprays can provide patients with an easy application and a rapid onset within minutes. In the last few years, some intraoral sprays such as Nicostar^{®}, Zonnic^{®} and Quickmist^{®} mouth sprays were developed by various companies.

Quickmist^{®} contains Poloxamer 407, which have been reported to exhibit mucoadhesive properties in gel formulations [Esra et al.: Rheological and mechanical properties of poloxamer mixtures as a mucoadhesive gel base. Pharmaceutical Development and Technology (2011)]. Present mucoadhesion studies of Quickmist^{®}, however, did not show any prolonged residence time of NCT on the intraoral mucosa (data shown in the experimental section below).

Zonnic^{®} and Nicostar^{®} do not contain any mucoadhesive polymers and the present data (in the experimental section below) did not show any prolonged residence time of NCT on the intraoral mucosa (data shown in the experimental section below).

So, the inventors of the present invention has found that all of the above mentioned products have a very low residence time of NCT in the order of a few minutes (studies presented herein). This result in a large amount of free NCT being washed off the intraoral mucosa towards the GIT, hereby causing side effects in the form of hick ups, stomach pain, irritation in the throat and oral cavity, and abdominal discomfort.

WO2008/037470 A1 discloses further oral spray compositions comprising nicotine.

None of the above mentioned prior art products disclose a residence time of NCT.

### Object and summary of the invention

The present invention was made in view of the prior art described above, and the object of the present invention is to provide a mucoadhesive oromucosal formulation according to the claims.

The invention relates to a liquid mouth spray device containing a liquid mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10.

It has surprisingly been found that it is possible to obtain a prolonged intraoral drug residence time for NCT. The prolonged residence time might show a good bioavailability and at the same time minimize the side effects. This is achieved by using a mucoadhesive oromucosal formulation comprising a nicotine complex, said nicotine complex comprising nicotine in ionic complex with at least one mucoadhesive water-soluble anionic polymer according to the present invention.

So far to the best of our knowledge there is no mucoadhesive liquid or gel formulation providing a prolonged intraoral drug residence time for NCT available, although it would be highly advantageous. According to the invention, the side effects, such as hick ups, stomach pain, irritation in the throat and intraoral cavity, and abdominal discomfort might be substantially lowered due to a prolonged residence time of the drug on the mucosa since the amount of free NCT being washed off the mucosa towards the GIT is reduced. Further, it should also lead to a good bioavailability in comparison to conventional NCT liquid formulations due to the prolonged residence time in the oral cavity.

Moreover, the invention also relates to a method of producing a liquid mouth spray device containing a liquid mucoadhesive oromucosal formulation comprising a nicotine complex and at least one preservative, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w, the method comprising the steps of:
- Mixing water with the at least one preservative
- Adding nicotine to the mixture
- Adding the at least one mucoadhesive water-soluble anionic polymer to the mixture
- Adjusting the pH of the formulation to between 8 and 10.

The invention further relates to a method of producing a liquid mouth spray device containing a liquid mucoadhesive oromucosal formulation comprising a nicotine complex and at least one preservative, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w, the method comprising the steps of:
- Mixing water with nicotine
- Adding the at least one mucoadhesive water-soluble anionic polymer to the mixture
- Adding the at least one preservative to the mixture
- Adjusting the pH of the formulation to between 8 and 10.

The invention further releates to a liquid or gel mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10, for use as a medicament.

The invention further releates to a liquid or gel mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10, for use in obtaining nicotine relief by administration to the oral cavity.

### Brief description of the drawings

In the following, preferred embodiments of the invention will be described referring to the figures, where;
Figure 1 shows a schematic presentation of the experimental setup for buccal residence time studies.
Figure 2 shows experimental set up for drug release studies.

### Detailed description

The inventors have found that it is possible to obtain a prolonged intraoral drug residence time for nicotine (NCT). The prolonged residence time might give a good bioavailability and at the same time minimize the side effects such as hick ups, stomach pain, irritation in the throat and oral cavity, and abdominal discomfort since the amount of free NCT being washed off the mucosa towards the GIT is reduced. Irritation in the throat and oral cavity is one of the most common side effects with conventional NCT mouth sprays (see the experimental section).

Accordingly, here is provided a liquid mouth spray device containing a liquid mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10.

Nicotine (NCT) is the main alkaloid found in tobacco and responsible for an addictive potential [Houezec et al.: Role of nicotine pharmacokinetics in nicotine addiction and nicotine replacement therapy: a review. The International Journal of Tuberculosis and Lung Disease, (2003)]. The chemical structure of NCT is illustrated below. NCT can be found in both its free base form as a liquid, or as an ionic complex in the form of a salt with a counter ion e.g., chloride ion (Cl⁻) or sulphate ion (HSO₄⁻).

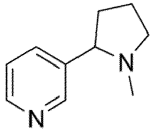

### The structure of NCT

In the pharmaceutical sciences the term mucoadhesion is used to describe when two surfaces, one of which is mucus or a mucous membrane and the other typically the surface of a drug delivery system, are held together for extended periods of time by interfacial forces.

By nicotine complex is meant the complex (loose association) formed between nicotine and the polymer, is meant the complex formed between the functional groups of nicotine (e.g. amine and pyridine) and the functional groups of the polymers (e.g. sulfate, alcohol and carboxylate etc.), is meant the non-covalent bond formed between nicotine and the polymer (e.g. ionic interaction and hydrogen bonding). The complexation between nicotine and the polymer will be affected by the environment (e.g. pH, solvent, concentrations and temperature).

Oromucosal formulation refers to preparations intended for administration to the oral cavity and/or the throat to obtain a local or systemic effect. The preparations are intended to be retained in the oral cavity by adhesion to the mucosal epithelium and may modify systemic drug absorption at the site of application.

By prolonged intraoral drug residence time for NCT is meant that the nicotine is present in the oral cavity for a longer time than shown for formulations which do not comprise a mucoadhesive nicotine complex formulation according to the present invention, as also shown in the experimental section. Further, prolonged intraoral drug residence time for NCT can also be meant as that the entire amount of nicotine is not washed off the oromucosa within the first 2 minutes, or within the first 5 minutes or within the first 10 minutes. By the present invention the NCT may be present in amount of more than 20%, such as 30%, such as 40%, such as 50%, such as 60%, such as 70%, such as 80%, such as 90% in the buccal cavity for more than 10 minutes. By the present invention the NCT may be present in amount of more than 20%, such as 30%, such as 40%, such as 50%, such as 60%, such as 70%, such as 80%, such as 85% in the buccal cavity for more than 20 minutes.

The water-soluble anionic polymer used in the present invention can be either synthetic or natural. The Water-soluble anionic polymer can be classified as linear, branched chain, crosslinked or network polymers.

The nicotine may be a free nicotine base in a liquid form.

The nicotine may be a nicotine salt.

The formulation may also comprise nicotine that is not in complex with at least one mucoadhesive water-soluble anionic polymer. In one embodiment a part of the nicotine present in the formulation is part of the nicotine complex and a part of the nicotine is free in the formulation meaning that it is not bound together with the at least one mucoadhesive water-soluble anionic polymer.

The concentration of the nicotine in the formulation may be between 0.1 w/w% and 10 w/w%, such as between 0.1 w/w% to 7 w/w%, such as between 0.25 w/w% and 5 w/w%, such as between 0.5 w/w% and 4 w/w%, such as between 0.5 w/w% and 3 w/w% such as between 0.5 w/w% and 2 w/w%, such as between 0.75 w/w% and 2 w/w%, such as between 1 w/w% and 2 w/w%, such as between 1.2 w/w% and 1.8 w/w%. In one embodiment the concentration of the nicotine in the formulation can be between 1.2 w/w% and 1.8 w/w%. A part of the nicotine present in the formulation may be part of the nicotine complex and a part of the nicotine may be free in the formulation meaning that it is not bound together with the at least one mucoadhesive water-soluble anionic polymer.

The at least one mucoadhesive water-soluble anionic polymer may be linear polymer(s).

The at least one mucoadhesive water-soluble anionic polymer may be branced chained polymer(s).

The at least one mucoadhesive water-soluble anionic polymer may be cross bound polyacrylic acid polymer(s).

The at least one mucoadhesive water-soluble anionic polymer may be selected from sulfated polysaccharides and/or anionic polysaccharides.

Polysaccharides are polymers of monosaccharides that can origin from plants, seaweed extracts (e.g. carrageenans), or microorganisms (e.g. xanthan gum). The polysaccharides can be anionic (charged) and/or sulfated.

The at least one mucoadhesive water-soluble anionic polymer may be xanthan gum(s). The at least one mucoadhesive water-soluble anionic polymer may be carrageenan(s). The at least one mucoadhesive water-soluble anionic polymer may be carbomer(s). In one embodiment the at least one mucoadhesive water-soluble anionic polymer may be selected from a combination of one or more of xanthan gum(s), carrageenan(s) and carbomer(s). In one embodiment the formulation comprises a combination of at least one xanthan gum and at least one carrageenan. In one embodiment the formulation comprises a combination of at least one xanthan gum and at least one carbomer. In one embodiment the formulation comprises a combination of at least one carrageenan and at least one carbomer.

Carrageenan is a family of linear sulphated polysaccharides. They are used for their gelling, thickening and stabilizing properties. There are three main varieties of carrageen, which differ in their degree of sulphation. Kappa-carrageen has one sulphate group per disaccharide, lota-carrageen two, and Lambda-carrageen three. Gelcarins are carrageenans and are linear polymers.

Gelcarin^{®} GP 379 NF is an Iota carrageenan, which can be used for gelling, thickening, and stabilizing applications [product brochure, www.fmcbiopolymer.com, 07.2017].

Xanthan gum is a polysaccharide used as an additive for thickening. It is composed of repeating pentasaccharide unites, comprising glucose, mannose and glucuronic acid. xanthan gums are branched chained polymers.

XANTURAL^{®} 180 is an 80-mesh (180 µm) xanthan gum product suitable for use as a pharmaceutical excipient. It prevents phase separation in suspensions and emulsions, and ensures products are free-flowing throughout their shelf-life. XANTURAL^{®} 180 is typically used in oral suspensions and syrups [https://www.ulprospector.com/en/na/PersonalCare/Detail/6576/375639/XANTURAL-180-XANTHAN-GUM].

Carbomer is a high molecular weight, crosslinked polyacrylic acid polymer, which is sold e.g. under the brand Carbopol^{®}. Carbopols are carbomers.

According to the invention, the concentration of the at least one mucoadhesive water-soluble anionic polymer in the formulation is between 0.1 w/w% and 4.0 w/w%.

Further, the concentration of the at least one mucoadhesive water-soluble anionic polymer in the formulation may be between 0.1 w/w% to 2 w/w%, such as between 0.25 w/w% and 2 w/w%, such as between 0.25 w/w% and 1 w/w%, such as between 0.25 w/w% and 0.75 w/w% such as between 0.5 w/w% and 2 w/w%, such as between 0.5 w/w% and 1.5 w/w%, such as between 0.6 w/w% and 1 w/w%, such as between 0.6 w/w% and 0.8 w/w%. In one embodiment the at least one mucoadhesive water-soluble anionic polymer in the formulation may be between 0.5 w/w% and 1.5 w/w%. In one embodiment the at least one mucoadhesive water-soluble anionic polymer in the formulation may be between 0.6 w/w% and 0.8 w/w%.

The formulation may further comprises at least one preservative. The at least one preservative may be selected from alcohols such as mono-alcohols, diols or polyalcohols. In one embodiment the at least one preservative is an alcohol selected from ethanol or propylene glycol. The at least one preservative may be chlorhexidine.

The formulation may be a liquid formulation.

The formulation may be in the form of a liquid mouth spray.

A liquid mouth spray, or oral spray, is a device containing a liquid, which is applied by spraying said liquid into the oral cavity, whereby the liquid (e.g., in the form of small drops or a mist) will distribute throughout the oral cavity.

The formulation may be in the form of an oromucosal gel.

A gel is a solid jelly like material that can have properties ranging from soft and weak to hard and tough. The definition of a gel is a substantially dilute crosslinked system, which exhibits no flow when in the steady state. By weight, a gel is mostly liquid, but it behaves like a solid due to the three-dimensional crosslinked network within said liquid. The crosslinking contributes to the adhesiveness of the gel. In this way, a gel is a dispersion of molecules of a liquid within a solid, in which the solid is the continuous phase and the liquid is the discontinuous phase. A oromucosal gel is a gel applied to the oral cavity.

When we refer to a liquid formulation, what is meant is a material being more fluid than the above described gel.

According to the invention, the pH of the formulation is between 8 and 10.

In one embodiment the pH of the formulation is between 8.5 and 10, such as between 8.5 and 9.5 or such as between 9 and 10. In one embodiment the pH of the formulation is between 8.5 and 9.5.

In one embodiment a buffer may be added to the formulation to adjust the pH. Buffers may assist in facilitating nicotine absorption. The buffer can be selected from the group consisting of potassium carbonate, potassium bicarbonate, sodium carbonate and sodium bicarbonate, potassium citrate and dipotassium phosphate, or mixtures thereof.

The formulation may be a solid formulation. In one embodiment the formulation may be in the form of a freeze dried powder. Freeze dried powder (lyophilized powder) is powder that had all, or most, of its water removed from a liquid compound, hereby creating a solid powder or cake. In one embodiment the formulation may be in the form of a lozenge. A lozenge is a small tablet intended for dissolving in the oral cavity.

By the present invention, is also provided a method of producing a liquid mouth spray device containing a liquid mucoadhesive oromucosal formulation comprising a nicotine complex and at least one preservative, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w, , the method comprising the steps of:
- Mixing water with the at least one preservative
- Adding nicotine to the mixture
- Adding the at least one mucoadhesive water-soluble anionic polymer to the mixture

- Adjusting the pH of the formulation to between 8 and 10.

By the present invention, is also provided a method of producing a liquid mouth spray device containing a liquid mucoadhesive oromucosal formulation comprising a nicotine complex and at least one preservative, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w, the method comprising the steps of:
- Mixing water with nicotine
- Adding the at least one mucoadhesive water-soluble anionic polymer to the mixture
- Adding the at least one preservative to the mixture

- Adjusting the pH of the formulation to between 8 and 10.

In one embodiment of the methods described above the pH of the formulation may be between 8.5 and 10, such as between 8.5 and 9.5 or such as between 9 and 10. In one embodiment the pH is as between 8.5 and 9.5.

A liquid mouth spray, or oral spray, is a device containing a liquid, which is applied by spraying said liquid into the oral cavity, whereby the liquid (e.g., in the form of small drops or a mist) will distribute throughout the oral cavity.

A gel is a solid jelly like material that can have properties ranging from soft and weak to hard and tough. The definition of a gel is a substantially dilute crosslinked system, which exhibits no flow when in the steady state. By weight, a gel is mostly liquid, but it behaves like a solid due to the three-dimensional crosslinked network within said liquid. The crosslinking contributes to the adhesiveness of the gel. In this way, a gel is a dispersion of molecules of a liquid within a solid, in which the solid is the continuous phase and the liquid is the discontinuous phase. A oral gel is a gel applied to the oral cavity.

The present invention also provides a liquid or gel mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10, for use as a medicament.

The present mucoadhesive oromucosal formulation (a NRT product) can be used in obtaining this nicotine satisfaction without smoking as the nicotine gets into the bloodstream by the use of the present mucoadhesive oromucosal formulation.

The present invention also provides a liquid or gel mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10, for use in obtaining nicotine relief by administration to the oral cavity.

Nicotine relief is another term also used to describe the feeling when the nicotine reaches the bloodstream and stimulates the brain and relief the symptoms of nicotine cravings. The present mucoadhesive oromucosal formulation (a NRT product) can be used in obtaining this nicotine relief without smoking as the nicotine gets into the bloodstream by the use of the present mucoadhesive oromucosal formulation.

Known mouth sprays used as NRT provide a very rapid increase in the plasma nicotine level (see example 6). By controlled release of nicotine into the oral cavity is meant that the rate of release of nicotine into the oral cavity is controlled and slower than with known NRT formulations (see example 7, 8, 9 and 10).

By prolonged intraoral drug residence time for NCT is meant that the nicotine is present in the oral cavity for a longer time than shown for mucoadhesive formulations which do not comprise a nicotine complex according to the present invention, as also shown in the experimental section. Further, prolonged intraoral drug residence time for NCT can also be meant as that the entire amount of nicotine is not washed off the oromucosa within the first 2 minutes, or within the first 5 minutes or within the first 10 minutes. By the present invention the NCT may be present in amount of more than 20%, such as 30%, such as 40%, such as 50%, such as 60%, such as 70%, such as 80%, such as 90% in the buccal cavity for more than 10 minutes. By the present invention the NCT may be present in amount of more than 20%, such as 30%, such as 40%, such as 50%, such as 60%, such as 70%, such as 80%, such as 85% in the buccal cavity for more than 20 minutes.

Reduced irritation in the throat and oral cavity is one of the most common side effects of using NRT. The present formulation reduce this side effect as the formulation will reside in the oral cavity by sticking to the mucosa in the buccal cavity thereby less of the formulation will be swallowed into the throat.

The nicotine may be a free nicotine base in a liquid form.

The nicotine may be a nicotine salt.

The at least one mucoadhesive water-soluble anionic polymer may be linear polymer(s). The at least one mucoadhesive water-soluble anionic polymer may be branced chained polymer(s). The at least one mucoadhesive water-soluble anionic polymer may be cross bound polyacrylic acid polymer(s). The at least one mucoadhesive water-soluble anionic polymer may be selected from sulfated polysaccharides and/or anionic polysaccharides. Polysaccharides are polymers of monosaccharides that can origin from plants, seaweed extracts (e.g. carrageenans), or microorganisms (e.g. xanthan gum). The polysaccharides can be anionic (charged) and/or sulfated. The at least one mucoadhesive water-soluble anionic polymer may be xanthan gum(s). The at least one mucoadhesive water-soluble anionic polymer may be carrageenan(s). The at least one mucoadhesive water-soluble anionic polymer may be carbomer(s). In one embodiment the at least one mucoadhesive water-soluble anionic polymer may be selected from a combination of one or more of xanthan gum(s), carrageenan(s) and carbomer(s).

Carrageenan is a family of linear sulphated polysaccharides. They are used for their gelling, thickening and stabilizing properties. There are three main varieties of carrageen, which differ in their degree of sulphation. Kappa-carrageen has one sulphate group per disaccharide, lota-carrageen two, and Lambda-carrageen three. Gelcarins are carrageenans and are linear polymers. Gelcarin^{®} GP-379 NF is an Iota carrageenan, which can be used for gelling, thickening, and stabilizing applications [product brochure, www.fmcbiopolymer.com, 07.2017].

Xanthan gum is a polysaccharide used as an additive for thickening. It is composed of repeating pentasaccharide unites, comprising glucose, mannose and glucuronic acid. Xanthan gums are branched chained polymers. XANTURAL^{®} 180 is an 80-mesh (180 µm) xanthan gum product suitable for use as a pharmaceutical excipient. It prevents phase separation in suspensions and emulsions, and ensures products are free-flowing throughout their shelf-life. XANTURAL^{®} 180 is typically used in oral suspensions and syrups [https://www.ulprospector.com/en/na/PersonalCare/Detail/6576/375639/XANTURAL-180-XANTHAN-GUM].

Carbomer is a high molecular weight, crosslinked polyacrylic acid polymer, which is sold e.g. under the brand Carbopol^{®}. Carbopols are carbomers.

In one embodiment of the invention the mucoadhesive oromucosal formulation according to the present invention may be administered to a human subject up to 80 times per day, such as up to 70 times per day, such as up to 60 times per day, such as up to 50 times per day or less. The patients should use the mucoadhesive oromucosal formulation formulated e.g. as a mouth spray, whenever needed to alleviate the nicotine craving. In one embodiment the mucoadhesive oromucosal formulation may be administered for up to 6 times every hour.

When describing the embodiments of the present invention, the combinations and permutations of all possible embodiments have not been explicitly described. Nevertheless, the mere fact that certain measures are recited in mutually different dependent claims or described in different embodiments does not indicate that a combination of these measures cannot be used to advantage. The present invention envisages all possible combinations and permutations of the described embodiments.

### Experimental:

The following non-limiting examples illustrate different variations of the present invention. The examples are meant for indicating the inventive concept; hence the mentioned examples should not be understood as exhaustive for the present invention.

The purpose of the below study was to investigate the residence time, and release rates of nicotine and mucoadhesive properties, sensory evaluation and of a number of compositions according to the invention.

### Materials:

(-)Nicotine base (Lot:1449194V, Sigma-Aldrich, Germany), Nicotine in accordance with USP, Aqua purificata (Millipore Ultrapure Water Purification System), Gelcarin GP 379 NF (Lot: 50124041-W1, FMC BioPolymer, USA), Xantural 180 (Lot:3E9161K, CP Kelco, DKSH Switzerland Ltd., Switzerland), Protanal CR 8223 (Lot: GQ9701103-W1, FMC BioPolymer, USA), SeaSpen PF NF (Lot: 60124041-W1, FMC BioPolymer, USA), Carbopol 974P NF (Lot:01014132001, Lubrizol, Belgium), Carbopol 71 G NF (Lot:000005771, Lubrizol, Belgium), Noveon AA-1 (Lot: 0100883650, Lubrizol, Belgium), Plasdone K-90 (Lot: 830833, Asland, USA), Mannucol LKX (Lot: GQ9105401-W1FMC BioPolymer, USA), Tragacanth Pulver (Lot:KNC0606714, Herba-Chemosan, Austria), Butylene glycol (Lot: STBF6778V, Sigma-Aldrich, Germany), Glycerol (Lot:BCBK1040V, Sigma-Aldrich, Germany), Ethanol (4521438970 Herba-Chemosan, Austria Germany), Ethanol in accordance with USP, Propylene glycol (Lot:STBD3558V Sigma-Aldrich, Germany), Propylene glycol in accordance with USP, Chlorhexidine acetate (Sigma-Aldrich, Germany), Chlorhexidine acetate in accordance with USP, Brilliant blue (E 133, Jacobi Decor GmbH, Germany), 5 M NaOH solution, 5 M HCl solution, 1 M NaOH solution, 1 M HCl solution.

### Preparation steps for placebo gels:

Aqua purificata (50 mL) was placed in a plastic beaker with a screw cap. Polymer (see Entry later in the experimental section) was slowly added to the water and the plastic beaker was closed with the cap before the dispersion was stirred for 48 h. The final pH was adjusted to pH = 8.5 by slowly adding solutions of hydrochloric acid (1 M) or sodium hydroxide (1 M). The final product was a clear or lightly turbid and slightly yellowish gel.

### Preparation steps for placebo gels with brilliant blue (blue color):

Aqua purificata (50 mL) was placed in a plastic beaker with a screw cap. Brilliant blue was added and dissolved by stirring. Polymer (see entry later in the experimental section) was slowly added to the water and the plastic beaker was closed with the cap before the dispersion was stirred for 48 h. The final pH was adjusted to pH = 8.5 by slowly adding solutions of hydrochloric acid (1 M) or sodium hydroxide (1 M). The final product was a clear or lightly turbid blue gel.

### Preparation steps for nicotine gels:

Aqua purificata (50 mL) was placed in a plastic beaker with a screw cap. Nicotine (750 µl) was dissolved in the water under constant stirring (500 rpm.). Afterwards, hydrochloric acid (5 M) was added dropwise to adjust the solution to reach a pH = 8.5. Polymer (see entry later in the experimental section) was then slowly added to the solution and the plastic beaker was closed with the cap before the dispersion was stirred for 48 h. The final pH was adjusted to pH = 8.5 by slowly adding solutions of hydrochloric acid (1 M) or sodium hydroxide (1 M). The final product was a clear or lightly turbid and slightly yellowish gel.

### Preparation steps for nicotine gels with chlorhexidine acetate:

Aqua purificata (50 mL) was placed in a plastic beaker with a screw cap. Chlorhexidine acetate (0.01 g) was dissolved in the water under constant stirring (500 rpm.) before nicotine (750 µl) was added to the solution. Afterwards, hydrochloric acid (5 M) was added dropwise to adjust the solution to pH = 8.5. Polymer (see entry later in the experimental section) was then slowly added to the solution and the plastic beaker was closed with the cap before the dispersion was stirred for 48 h. The final pH was adjusted to pH = 8.5 by slowly adding solutions of hydrochloric acid (1 M) or sodium hydroxide (1 M). The final product was a clear or lightly turbid and slightly yellowish gel.

### Preparation steps for nicotine gels with alcohols; Formulation I:

Aqua purificata (35 mL) and alcohol (15 mL, see entry later in the experimental section) was placed in a plastic beaker with a screw cap. Nicotine (750 µl) was dissolved in the water under constant stirring (500 rpm.). Afterwards, hydrochloric acid (5 M) was added dropwise to adjust the solution to pH = 8.5. Polymer (see entry later in the experimental section) was then slowly added to the solution and the plastic beaker was closed with the cap before the dispersion was stirred for 48 h. The final pH was adjusted to pH = 8.5 by slowly adding solutions of hydrochloric acid (1 M) or sodium hydroxide (1 M). The final product was a clear or lightly turbid and slightly yellowish gel.

### Preparation steps for nicotine gels with alcohols; Formulation II:

Aqua purificata (35 mL) was placed in a plastic beaker with a screw cap. Nicotine (750 µl) was dissolved in the water under constant stirring (500 rpm.). Afterwards, hydrochloric acid (5 M) was added dropwise to adjust the solution to pH = 8.5. Polymer (see entry later in the experimental section) was then slowly added to the solution and the plastic beaker was closed with the cap before the dispersion was stirred for 48 h. Alcohol (15 mL, see entry later in the experimental section) was slowly added and the gel before it was stirred for additional 12 h. The final pH was adjusted to pH = 8.5 by slowly adding solutions of hydrochloric acid (1 M) or sodium hydroxide (1 M). The final product was a clear or lightly turbid and slightly yellowish gel.

### In vitro screening of mucoadhesive properties:

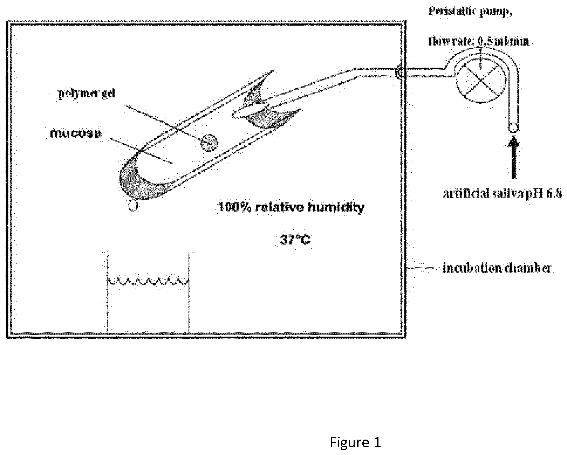

Porcine buccal mucosa freshly obtained from a local slaughter house was mounted on a half-pipe and placed in an incubating cupboard with 100% humidity and a temperature of 37°C at an angle of 45° and rinsed with artificial saliva as shown in Fig. 1. Artificial saliva adjusted to pH 6.8 contains potassium chloride 1.2 (g/l), sodium chloride 0.85 (g/l), magnesium chloride 0.05 (g/l), calcium chloride 0.13 (g/l), di-potassium hydrogen orthophosphate 0.13 (g/l) according to Preethan et al (Preetha A. and Banerjee R., Comparison of Artificial Saliva Substitutes, Trends Biomater. Artif. Organs, 18, 2005, 178-186.)

After 30 min incubation time the rinsing was stopped and 0.3 ml of the gels were placed onto the buccal mucosa. Thereafter, rinsing with artificial saliva having a pH of 6.8 was immediately started and the first samples were collected after 10 min. During the whole measurement, the temperature of the incubation chamber was kept at 37°C. A constant flow rate of 0.5 ml/min was provided by using a peristaltic pump (Ismatec IPC, Wertheim, Germany). During the incubation, fractions of artificial saliva (5 ml or 10 ml depending on the respective incubation time) were collected after 10, 20, 40, and 60 minutes and the amount of NCT were quantified via HPLC. At the end of each experiment the remaining gel was scraped off and also quantified, whereby, 0.3 ml of NCT containing gels with the proper polymer diluted with 5 or 10 ml of artificial saliva served as 100% control value and all other values were quantified in reference to them. Prior to HPLC analysis, samples collected from mucoadhesion studies were diluted with icecold ACN or MetOH in a ratio 1:2 (V:V) in order to precipitate the polymer traces and subsequently centrifuged at 12500 rpm for 10 min.

### Drug release studies in artificial saliva:

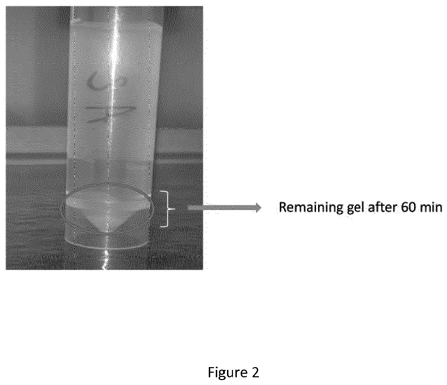

The drug release studies in artificial saliva were carried out employing the following procedure:
0.1 ml of gels containing NCT was placed onto the bottom of a plastic tube. Afterwards, 5 ml preheated (37°C) artificial saliva pH 5.0 or 6.8 were slowly poured over the gel. Thereafter, the tube was incubated at 37 °C under continuous shaking at 300 rpm. Samples of 20 µl were removed after 5, 10, 15, 30, 45 and 60 min which were diluted with icecold ACN or MetOH in a ratio of 1:2 (V:V), centrifuged at 12500 rpm for 10 min and analyzed via HPLC. Within this experimental setup, 0.1 ml of gel formulation - dissolved in 5 ml of buffer and stirred vigorously by using a Vortex mixer for 5 min - served as 100% control. The experimental setup with the remaining gels after 60 min can be seen in Fig. 2. As a reference, a NCT solution was prepared in a mixture of water and glycerol (50-50% V/V) at pH 8.5 and the release studies were performed using this NCT solution as well.

### Description of in vivo experiments:

### Mucoadhesive evaluation:

Placebo gels (entry 100-103 see entries later in the experimental section) with brilliant blue was prepared according to the described method and transferred to a standard spray device. The reference mouth spray (entry 99) was prepared by adding brilliant blue (0.01 g) to a mixture of aqua purificata (35 mL) and glycerol (15 mL). The obtained blue reference solution was transferred to a standard spray device.

The test was performed by spraying the formulation (~70 µl) in quest onto the buccal surface of a test person. The evaluation was performed by estimating the amount of blue color on the buccal surface (Table 11). From Table 11 it is clear that the residence time of the mouth sprays (entry 100-103) containing polymer is much longer than for the reference mouth spray (entry 99).

### Sensory evaluation - mouthfeel and irritation:

The gels (entry 104-111, see entries later in the experimental section) was prepared according to the described method and transferred to a standard spray device. The reference mouth spray was Quickmist^{®}. The test was performed by applying one pump (~70 µl) of the spray in question onto the buccal surface of five test persons. The evaluation was performed by estimating the mouthfeel (Table 12) and irritation in the mouth and throat (Table 13). From Table 12 it is clear that the mouthfeel of the mouth sprays (entry 104-111) is similar to water. From Table 13 it is clear that the irritation experienced in the mouth is comparable or less for the developed mouth sprays (entry 104-111) compared to the reference mouth spray. The irritation experienced in the throat is considerably less for the developed mouth sprays (entry 104-111) compared to the reference mouth spray.

### Polymer concentration:

| Entry | Gelcarin GP 379 (g) | Polymer (%) | H₂O (ml) | Nicotine (µl) | pH |
|---|---|---|---|---|---|
| 1 | 0.05 | 0.1 | 50 | 750 | 8.5 |
| 2 | 0.10 | 0.2 | 50 | 750 | 8.5 |
| 3 | 0.15 | 0.3 | 50 | 750 | 8.5 |
| 4 | 0.20 | 0.4 | 50 | 750 | 8.5 |
| 5 | 0.25 | 0.5 | 50 | 750 | 8.5 |
| 6 | 0.30 | 0.6 | 50 | 750 | 8.5 |
| 7 | 0.35 | 0.7 | 50 | 750 | 8.5 |
| 8 | 0.375 | 0.75 | 50 | 750 | 8.5 |
| 9 | 0.40 | 0.8 | 50 | 750 | 8.5 |
| 10 | 0.45 | 0.9 | 50 | 750 | 8.5 |
| 11 | 0.50 | 1.0 | 50 | 750 | 8.5 |
| 12 | 0.55 | 1.1 | 50 | 750 | 8.5 |
| 13 | 0.60 | 1.5 | 50 | 750 | 8.5 |
| 14 | 1.0 | 2.0 | 50 | 750 | 8.5 |

| Entry | Xantural 180 (g) | Polymer (%) | H₂O (ml) | Nicotine (µl) | pH |
|---|---|---|---|---|---|
| 15 | 0.05 | 0.1 | 50 | 750 | 8.5 |
| 16 | 0.10 | 0.2 | 50 | 750 | 8.5 |
| 17 | 0.15 | 0.3 | 50 | 750 | 8.5 |
| 18 | 0.20 | 0.4 | 50 | 750 | 8.5 |
| 19 | 0.25 | 0.5 | 50 | 750 | 8.5 |
| 20 | 0.30 | 0.6 | 50 | 750 | 8.5 |
| 21 | 0.35 | 0.7 | 50 | 750 | 8.5 |
| 22 | 0.375 | 0.75 | 50 | 750 | 8.5 |
| 23 | 0.40 | 0.8 | 50 | 750 | 8.5 |
| 24 | 0.45 | 0.9 | 50 | 750 | 8.5 |
| 25 | 0.50 | 1.0 | 50 | 750 | 8.5 |
| 26 | 0.55 | 1.1 | 50 | 750 | 8.5 |
| 27 | 0.60 | 1.5 | 50 | 750 | 8.5 |

| Entry | Carbopol 974 G (g) | Polymer (%) | H₂O (ml) | Nicotine (µl) | pH |
|---|---|---|---|---|---|
| 28 | 0.125 | 0.25 | 50 | 750 | 8.5 |
| 29 | 0.25 | 0.5 | 50 | 750 | 8.5 |
| 30 | 0.50 | 1.0 | 50 | 750 | 8.5 |

| Entry | Carbopol 71 G (g) | Polymer (%) | H₂O (ml) | Nicotine (µl) | pH |
|---|---|---|---|---|---|
| 31 | 0.25 | 0.5 | 50 | 750 | 8.5 |
| 32 | 0.50 | 1.0 | 50 | 750 | 8.5 |

| Entry | Noveon AA-1 (g) | Polymer (%) | H₂O (ml) | Nicotine (µl) | pH |
|---|---|---|---|---|---|
| 33 | 0.25 | 0.5 | 50 | 750 | 8.5 |
| 34 | 0.50 | 1.0 | 50 | 750 | 8.5 |

| Entry | Sodium Hyarulonate (g) | Polymer (%) | H₂O (ml) | Nicotine (µl) | pH |
|---|---|---|---|---|---|
| 35 | 0.50 | 1.0 | 50 | 2500 | 8.5 |

| Entry | Methocel K100 (g) | Polymer (%) | H₂O (ml) | Nicotine (µl) | pH |
|---|---|---|---|---|---|
| 36 | 1.5 | 3 | 50 | 2500 | 8.5 |

| Entry | Tragacantha (g) | Polymer (%) | H₂O (ml) | Nicotine (µl) | pH |
|---|---|---|---|---|---|
| 37 | 0.50 | 1.0 | 50 | 2500 | 8.5 |

| Entry | Plasdone K-90 (g) | Polymer (%) | H₂O (ml) | Nicotine (µl) | pH |
|---|---|---|---|---|---|
| 38 | 5.0 | 10 | 50 | 2500 | 8.5 |

| Entry | SeaSpen (g) | Polymer (%) | H₂O (ml) | Nicotine (µl) | pH |
|---|---|---|---|---|---|
| 39 | 0.50 | 1.0 | 50 | 2500 | 8.5 |
| 40 | 1.0 | 2.0 | 50 | 2500 | 8.5 |

| Entry | Protanal CR8223 (g) | Polymer (%) | H₂O (ml) | Nicotine (µl) | pH |
|---|---|---|---|---|---|
| 41 | 1.5 | 3.0 | 50 | 2500 | 8.5 |
| 42 | 2.0 | 4.0 | 50 | 2500 | 8.5 |

| Entry | Mannucol LKX (g) | Polymer (%) | H₂O (ml) | Nicotine (µl) | pH |
|---|---|---|---|---|---|
| 43 | 1.0 | 2.0 | 50 | 2500 | 8.5 |

| Entry | PAA 450 kDa (g) | Polymer (%) | H₂O (ml) | Nicotine (µl) | pH |
|---|---|---|---|---|---|
| 44 | 0.25 | 0.5 | 50 | 2500 | 8.5 |
| 45 | 0.50 | 1.0 | 50 | 2500 | 8.5 |
| 46 | 1.0 | 2.0 | 50 | 2500 | 8.5 |
| 47 | 2.0 | 4.0 | 50 | 2500 | 8.5 |

| Entry | PAA 1250 kDa (g) | Polymer (%) | H₂O (ml) | Nicotine (µl) | pH |
|---|---|---|---|---|---|
| 48 | 0.25 | 0.5 | 50 | 2500 | 8.5 |
| 49 | 0.50 | 1.0 | 50 | 2500 | 8.5 |
| 50 | 1.0 | 2.0 | 50 | 2500 | 8.5 |

| Entry | PAA 3000 kDa (g) | Polymer (%) | H₂O (ml) | Nicotine (µl) | pH |
|---|---|---|---|---|---|
| 51 | 0.25 | 0.5 | 50 | 2500 | 8.5 |
| 52 | 0.50 | 1.0 | 50 | 2500 | 8.5 |

| Entry | Gelcarin GP 812 NF (g) | Polymer (%) | H₂O (ml) | Nicotine (µl) | pH |
|---|---|---|---|---|---|
| 53 | 0.50 | 1.0 | 50 | 2500 | 8.5 |

Entry 38, comprising 10% polymer, does not fall under the scope of the claims.

### Preservative:

| Entry | Gelcarin GP 379 (g) | Polymer (%) | H₂O (ml) | PG (ml) | EtOH (ml) | Chlorhexidine acetate (mg) | Nicotine (µl) | pH |
|---|---|---|---|---|---|---|---|---|
| 54 | 0.75 | 1.5 | 50 | - | - | - | 750 | 8.5 |
| 55 | 0.75 | 1.5 | 35 | 15 | - | - | 750 | 8.5 |
| 56 | 0.75 | 1.5 | 35 | - | 15 | - | 750 | 8.5 |
| 57 | 0.75 | 1.5 | 50 | - | - | 10 | 750 | 8.5 |

| Entry | Xantural 180 (g) | H₂O (ml) | PG (ml) | EtOH (ml) | Isopropyl alcohol (ml) | Butylene glycol (ml) | Glycerol (ml) | Chlorhexidine acetate (mg) | NCT (µl) | pH |
|---|---|---|---|---|---|---|---|---|---|---|
| 58 | 0.5 | 50 | - | - | - | - | - | - | 750 | 8.5 |
| 59 | 0.5 | 35 | 15 | - | - | - | - | - | 750 | 8.5 |
| 60 | 0.5 | 35 | - | 15 | - | - | - | - | 750 | 8.5 |
| 61 | 0.5 | 35 | - | - | 15 | - | - | - | 750 | 8.5 |
| 62 | 0.5 | 35 | - | - | - | 15 | - | - | 750 | 8.5 |
| 63 | 0.5 | 35 | - | - | - | - | 15 | - | 750 | 8.5 |
| 64 | 0.5 | 50 | - | - | - | - | - | 10 | 750 | 8.5 |

| Entry | Carbopol 974 G (g) | Polymer (%) | H₂O (ml) | PG (ml) | EtOH (ml) | Chlorhexidine acetate (mg) | Nicotine (µl) | pH |
|---|---|---|---|---|---|---|---|---|
| 65 | 0.125 | 0.25 | 50 | - | - | - | 750 | 8.5 |
| 66 | 0.125 | 0.25 | 35 | 15 | - | - | 750 | 8.5 |
| 67 | 0.125 | 0.25 | 35 | - | 15 | - | 750 | 8.5 |
| 68 | 0.25 | 0.5 | 35 | 15 | - | - | 750 | 8.5 |

| Entry | Noveon AA-1 (g) | Polymer (%) | H₂O (ml) | PG (ml) | EtOH (ml) | Chlorhexidine acetate (mg) | Nicotine (µl) | pH |
|---|---|---|---|---|---|---|---|---|
| 69 | 0.125 | 0.25 | 50 | - | - | - | 750 | 8.5 |
| 70 | 0.125 | 0.25 | 35 | 15 | - | - | 750 | 8.5 |
| 71 | 0.125 | 0.25 | 35 | - | 15 | - | 750 | 8.5 |
| 72 | 0.25 | 0.5 | 50 | - | - | - | 750 | 8.5 |
| 73 | 0.25 | 0.5 | 35 | 15 | - | - | 750 | 8.5 |
| 74 | 0.25 | 0.5 | 35 | - | 15 | - | 750 | 8.5 |

| Entry | PAA 1250 kDa (g) | Polymer (%) | H₂O (ml) | PG (ml) | EtOH (ml) | Chlorhexidine acetate (mg) | Nicotine (µl) | pH |
|---|---|---|---|---|---|---|---|---|
| 75 | 0.25 | 0.5 | 50 | - | - | - | 2500 | 8.5 |
| 76 | 0.25 | 0.5 | 35 | 15 | - | - | 2500 | 8.5 |
| 77 | 0.25 | 0.5 | 35 | - | 15 | - | 2500 | 8.5 |
| 78 | 0.5 | 1.0 | 50 | - | - | - | 2500 | 8.5 |
| 79 | 0.5 | 1.0 | 35 | 15 | - | - | 2500 | 8.5 |
| 80 | 0.5 | 1.0 | 35 | - | 15 | - | 2500 | 8.5 |

### Polymer blend:

| Entry | Gelcarin GP 379 (g) | Polymer (g) | H₂O (ml) | Nicotine (µl) | pH |
|---|---|---|---|---|---|
| 81 | 0.375 | CP 974 (0.25) | 50 | 750 | 8.5 |
| 82 | 0.25 | CP 974 (0.1) | 50 | 750 | 8.5 |
| 83 | 0.375 | Xantural 180 (0.25) | 50 | 750 | 8.5 |
| 84 | 0.25 | Xantural 180 (0.25) | 50 | 750 | 8.5 |
| 85 | 0.375 | Xantural 180 (0.5) | 50 | 750 | 8.5 |
| 86 | 0.375 | Xantural 180 (1.0) | 50 | 750 | 8.5 |
| 87 | 0.375 | Xantural 180 (0.25) | 50 | 750 | 8.5 |

| Entry | Carbopol 974 G (g) | Polymer (g) | H₂O (ml) | Nicotine (µl) | pH |
|---|---|---|---|---|---|
| 88 | 0.25 | Protanal CR8223 (0.5) | 50 | 750 | 8.5 |

| Entry | Xantural 180 (g) | Polymer (g) | H₂O (ml) | Nicotine (µl) | pH |
|---|---|---|---|---|---|
| 89 | 0.5 | CP 974 (0.25) | 50 | 750 | 8.5 |
| 90 | 0.25 | CP 974 (0.25) | 50 | 750 | 8.5 |

### Nicotine concentrations:

| Entry | Polymer (g) | Polymer (%) | H₂O (ml) | Nicotine (µl) | pH |
|---|---|---|---|---|---|
| 91 | Gelcarin 379 (0.25) | 0.5 | 50 | 2500 | 8.5 |
| 92 | Gelcarin 379 (0.5) | 1.0 | 50 | 2500 | 8.5 |
| 93 | Gelcarin 379 (0.25) | 0.5 | 50 | 750 | 8.5 |
| 94 | Gelcarin 379 (0.5) | 1.0 | 50 | 750 | 8.5 |
| 95 | Carbopol 974 (0.25) | 0.5 | 50 | 2500 | 8.5 |
| 96 | Carbopol 974 (0.25) | 0.5 | 50 | 750 | 8.5 |

### Formulation I and II (F I and F II):

| Entry | Formulation method | Gelcarin 379 (g) | Polymer (%) | H₂O (ml) | PG (ml) | Nicotine (µl) | pH |
|---|---|---|---|---|---|---|---|
| 97 | F I | 0.375 | 1.5 | 35 | 15 | 750 | 8.5 |
| 98 | F II | 0.375 | 1.5 | 35 | 15 | 750 | 8.5 |

### Formulations containing brilliant blue:

| Entry | Polymer (g) | Polymer (%) | H₂O (ml) | Brilliant blue (mg) | Ethanol(E)/ Glycerol(G) | pH |
|---|---|---|---|---|---|---|
| 99 | no, 30% glycerol | - | 35 | 10 | 15 (G) | 8.5 |
| 100 | Xanthan Gum (0.25) | 0.5 | 50 | 10 | | 8.5 |
| 101 | Xanthan Gum (0.5) | 1.0 | 50 | 10 | | 8.5 |
| 102 | Gelcarin 379 (0.5) | 1.0 | 50 | 10 | | 8.5 |
| 103 | Carbopol 974 (0.125) | 0.25 | 40 | 10 | 10 (E) | 8.5 |

### Formulations for sensory tests:

| Entry | Polymer (g) | Polymer (%) | H₂O (ml) | Ethanol (ml) | Propylene glycol (ml) | Chlorhexidine acetate (mg) | NCT (µL) | pH |
|---|---|---|---|---|---|---|---|---|
| 104 | Xantural 180 (0.375) | 0.75 | 50 | - | - | - | 750 | 8.5 |
| 105 | Xantural 180 (0.375) | 0.75 | 35 | 15 | | - | 750 | 8.5 |
| 106 | Xantural 180 (0.375) | 0.75 | 35 | - | 15 | - | 750 | 8.5 |
| 107 | Xantural 180 (0.375) | 0.75 | 50 | - | - | 10 | 750 | 8.5 |
| 108 | Gelcarin 379 (0.375) | 0.75 | 50 | - | - | - | 750 | 8.5 |
| 109 | Gelcarin 379 (0.375) | 0.75 | 35 | 15 | | - | 750 | 8.5 |
| 110 | Gelcarin 379 (0.375) | 0.75 | 35 | - | 15 | - | 750 | 8.5 |
| 111 | Gelcarin 379 (0.375) | 0.75 | 50 | - | - | 10 | 750 | 8.5 |

### Results from in vitro buccal residence time experiments:

### Reference Example 1:

The buccal residence time of the nicotine was evaluated as earlier described. The results of residence time studies of the reference mouth sprays (Quickmist^{®}, Zonnic^{®}, Nicostar^{®}) are shown in Table 1. These products did not show mucoadhesive properties as the entire amount of nicotine was washed off the mucosa within the first 10 min.

The tested Quickmist^{®} mounth spray contains Propylene glycol, anhydrous ethanol trometamol, poloxamer 407, glycerol, sodium hydrogen carbonate, levomenthol, mint flavour, cooling flavor, sucralose, acesulfame potassium, hydrochloric acid and purified water.

The tested Zonnic^{®} mounth spray contains nicotine (one packaged spray contains 200 doses and contains 1 mg nicotine), sucralose, ethanol, glycerin, mint flavors, potassium dihydrogen phosphate, sodium hydroxide and water.

The tested Nicostar^{®} mounth spray contains water, glycerine, acerola exstract, liquorice exstract, coffee aroma, nicotine (0.081 to 0.099 %wt), citric acid, potassium sorbate, sodium benzoate, ginger extract, sucralose, pegylated hydrogenated castor oil and epigallocatechin gallate.

**Table 1: Remaining NCT on the mucosa in function of time. Indicated values are means of at least 3 samples ± SD.**

| Entry | Time (min) | | | | | |
|---|---|---|---|---|---|---|
| | 10 | 20 | 40 | 60 | 80 | 100 |
| Quickmist | 2±1 | 0 | - | - | - | - |
| Zonnic | 3±1 | 0 | - | - | - | - |
| Nicostar | 1±1 | 0 | - | - | - | - |

### Example 2:

The buccal residence time of the nicotine was evaluated as earlier described. The mucoadhesive properties of Xantural 180 gels with various concentrations (0.5, 1.0 and 1.5%) of the polymer are presented in Table 2. Using Xantural 180 a prolonged residence time was detected. A clear correlation between residence time and polymer concentration could be observed, namely, an increasing content of polymer resulted in an increasing residence time of nicotine.

**Table 2: Mucoadhesive property of selected nicotine containing gels (Xantural 180). Indicated values are means of at least 3 samples ± SD.**

| Entry | Time (min) | | | |
|---|---|---|---|---|
| | 10 | 20 | 40 | 60 |
| 19 | 22±12 | 10±5 | 3±1 | 0 |
| 25 | 92±4 | 85±6 | 64±14 | 54±8 |
| 27 | 88±4 | 80±5 | 69±6 | 61±5 |

### Example 3:

### (Xantural 180 1.0% + (without preservatives, EtOH, PG, chlorhexidine)

The effect of different preservatives onto the residence time was assessed. As can be seen in Table 3 the presence of preservatives (EtOH, propylene glycol or chlorhexidine acetate) decreased the residence time comparing to the reference gel without preservatives.

**Table 3: Mucoadhesive property of Xantural 180 based gels containing selected preservatives. Indicated values are means of at least 3 samples ± SD.**

| Entry | Time (min) | | | |
|---|---|---|---|---|
| | 10 | 20 | 40 | 60 |
| 58 | 92±4 | 85±6 | 64±14 | 54±8 |
| 60 | 64±34 | 42±28 | 26±17 | 14±9 |
| 59 | 71±23 | 57±29 | 43±30 | 31±23 |
| 64 | 66±15 | 25±6 | 16±6 | 10±4 |

### Example 4:

### (Xantural 180 0.50% + (without preservatives, Carbopol 974 0.5% and Gelcarin GP-379 NF 0.75%)

The effect of different polymer blends onto the residence time was assessed. As can be seen in Table 4, the blend of different polymers effected the residence time. The blends gave an increased residence time compared to the pure Xantural 180 gel.

**Table 4: Mucoadhesive property of Xantural 180 gels containing selected polymer blends. Indicated values are means of at least 3 samples ± SD.**

| Entry | Time (min) | | | |
|---|---|---|---|---|
| | 10 | 20 | 40 | 60 |
| 19 | 22±12 | 11±5 | 3±1 | 0 |
| 90 | 82±7 | 64±19 | 40±29 | 29±27 |
| 87 | 84±2 | 57±15 | 32±19 | 19±15 |

### Example 5:

### Gelcarin GP-379 NF 1.5% + (without preservatives, PG F I and PG F II)

The effect of different formulation methods was assessed. As can be seen in Table 5., the different formulations methods I and II affected the residence time. Formulation method II gave an increased residence time compared to formulation method I.

**Table 5: Mucoadhesive property of Gelcarin GP-379 NF gels containing propylene glycol formulated by two different methods. Indicated values are means of at least 3 samples ± SD.**

| Entry | Time (min) | | | |
|---|---|---|---|---|
| | 10 | 20 | 40 | 60 |
| 8 | 93±5 | 87±6 | 78± | 70±15 |
| 97 | 32±9 | 10±2 | 4±2 | 1±1 |
| 98 | 84±7 | 68±6 | 41±2 | 25±5 |

### Results from nicotine release experiments:

### Reference Example 6:

Reference NCT solution containing 50% glycerol. Release buffer pH 6.8.

Release of NCT from a NCT reference solution containing 50% glycerol was evaluated. As can be seen in Table 6, the whole amount of NCT was released within 30 min, furthermore, after 5 min 79% of NCT was released.

**Table 6: Release profile of NCT from NCT reference solution. Indicated values are means of at least 3 samples ± SD. Release buffer pH 6.8.**

| Entry | Time (min) | | | |
|---|---|---|---|---|
| | 5 | 10 | 15 | 30 |
| Reference solution | 79±7 | 84±13 | 84±2 | 95±10 |

### Example 7:

Xantural 180 (0.5, 1.0 and 1.5%) Release buffer pH 6.8.

The effect of concentration of the polymer was evaluated onto the rate of release. As can be seen in Table 7, an increasing amount of Xantural 180 resulted in a slower rate of release. In addition, the release rate was tremendously decreased comparing to the reference solution (Table 6).

**Table 7: Release profile of NCT from Xantural gels (0.5%, 1%, 1.5%). Indicated values are means of at least 3 samples ± SD. Release buffer pH 6.8.**

| Entry | Time (min) | | | | | |
|---|---|---|---|---|---|---|
| | 10 | 20 | 30 | 40 | 50 | 60 |
| 19 | 42±6 | 58±7 | 59±1 | 67±6 | 73±10 | 76±7 |
| 25 | 34±2 | 46±3 | 48±3 | 53±7 | 58±7 | 60±5 |
| 27 | 9±1 | 13±2 | 21±1 | 38±4 | 44±6 | 49±6 |

### Example 8:

### (Xantural 180 (0.5%) release medium: pH = 5.0 and 6.8; and Xantural 180 (1.5%) release medium :pH = 5.0 and 6.8).

The pH of the release medium was assessed onto the rate of release. Therefore, the release studies were performed in a buffer at pH 5.0 and at pH 6.8. As can be seen in Table 8 , the release was comparatively faster in release medium exhibiting a higher pH value (pH 6.8). This effect was observed in case of both concentrations.

**Table 8: Release profile of NCT from Xantural gels (0.5%, 1.5%) in release buffer exhibiting two different pH values. Indicated values are means of at least 3 samples ± SD.**

| Entry | Time (min) | | | | | |
|---|---|---|---|---|---|---|
| | 10 | 20 | 30 | 40 | 50 | 60 |
| 19 pH 5.0 | 21±6 | 31±7 | 40±1 | 46±6 | 54±10 | 61±7 |
| 19 pH 6.8 | 42±6 | 58±7 | 59±1 | 67±6 | 73±10 | 76±7 |
| 27 pH 5.0 | 5±1 | 10±1 | 14±3 | 24±5 | 32±5 | 41±7 |
| 27 pH 6.8 | 9±1 | 13±2 | 21±1 | 38±4 | 44±6 | 49±6 |

### Example 9:

Xantural 180 (1.0%) + (without preservatives, PG, ethanol, isopropyl alcohol, butylene glycol, glycerol, chlorhexidine acetate ) Release buffer pH 6.8.

The presence of selected preservatives was evaluated onto the rate of release of NCT from Xantural gels. Gels containing polyvalent alcohols (glycerol, PG, butylene glycol) showed a slow release of NCT from the gels, whereby monovalent alcohols (ethanol, isopropyl alcohol) did not change the release kinetic dramatically. In addition, chlorhexidine acetate had only a minor effect onto the rate of release of NCT from the gels.

**Table 9: Release profile of NCT from Xantural gel (1.0%) containing selected preservatives. Indicated values are means of at least 3 samples ± SD. Release buffer pH 6.8.**

| Entry | Time (min) | | | | | |
|---|---|---|---|---|---|---|
| | 10 | 20 | 30 | 40 | 50 | 60 |
| 58 | 34±2 | 46±3 | 48±3 | 53±7 | 58±7 | 60±5 |
| 59 | 4±1 | 3±1 | 3±1 | 4±1 | 4±1 | 4±1 |
| 60 | 19±3 | 28±1 | 32±1 | 38±3 | 44±4 | 48±5 |
| 61 | 16±4 | 25±2 | 32±2 | 34±2 | 37±2 | 40±3 |
| 62 | 3±1 | 5±1 | 5±1 | 7±1 | 9±1 | 9±1 |
| 63 | 4±2 | 4±2 | 4±2 | 4±2 | 4±2 | 4±2 |
| 64 | 7±3 | 17±2 | 26±4 | 31±4 | 39±3 | 50±5 |

### Example 10:

Carbopol 974 0.5% + (Protanal CR 8233 1%, Gelcarin GP-379 NF 0.75 % or Xantural 0.5%). Release buffer pH 6.8.

Selected Carbopol 974 based polymer blends were tested regarding release of NCT from the gels. In this process the fastest release was observed in the respect of Gelcarin GP-379 NF containing gel, whereas Protonal CR 8233 containing gel exhibited the slowest release rate.

**Table 10: Release profile of NCT from CP 974 (0.5%) based gels containing selected polymer blends. Indicated values are means of at least 3 samples ± SD. Release buffer pH 6.8.**

| Entry | Time (min) | | | | | |
|---|---|---|---|---|---|---|
| | 10 | 20 | 30 | 40 | 50 | 60 |
| 88 | 5±1 | 12±3 | 20±4 | 26±3 | 35±6 | 41±6 |
| 81 | 14±4 | 30±10 | 41±11 | 47±12 | 56±15 | 68±6 |
| 90 | 13±5 | 18±6 | 34±8 | 38±9 | 48±4 | 51±5 |

### Results of in vivo residence time experiments

### Example 11:

Evaluation of residence time in vivo.

The test was performed by spraying the formulation (~70 µl) in quest onto the buccal surface of a test person. The evaluation was performed by estimating the amount of blue color on the buccal surface (Table 11). From Table 11 it is clear that the residence time of the mouth sprays (entry 100-103) containing polymer is much longer than for the reference mouth spray (entry 99).

**Table 11: Evaluation of the amount of blue color on buccal mucosa: +++++ = high; + = low.**

| Entry | Time (min) | | | |
|---|---|---|---|---|
| | 0 | 5 | 10 | 15 |
| 99 | +++++ | - | - | - |
| 100 | +++++ | ++++ | +++ | + |
| 101 | +++++ | ++++ | +++ | ++ |
| 102 | +++++ | ++++ | +++ | ++ |
| 103 | +++++ | ++ | + | - |

### Results of sensory evaluation

### Example 12:

Mouthfeel and irritation of the mouth sprays (104-111) and Quickmist^{®}.

The effect of different formulation methods was assessed. From Table 12 it is clear that the mouthfeel of the mouth sprays (entry 104-111) is like water. From Table 13 it is clear that the irritation experienced in the mouth is comparable or less for the developed mouth sprays (entry 104-111) compared to the reference mouth spray (Quickmist^{®}). The irritation experienced in the throat is considerably less for the developed mouth sprays (entry 104-111) compared to the reference mouth spray (Quickmist^{®}).

**Table 12: Mouthfeel of the mouth sprays**

| Entry | Mouthfeel |
|---|---|
| 104 | +++++ |
| 105 | +++++ |
| 106 | N/A |
| 107 | N/A |
| 108 | +++++ |
| 109 | ++++ |
| 110 | +++++ |
| 111 | N/A |
| Quickmist^{®} | +++++ |

| | |
|---|---|
| Evaluation: +++++ = like water; + = unacceptable gel like | |

**Table 13 : Irritation of the mouth sprays**

| Entry | Mouth | Throat |
|---|---|---|
| 104 | +++ | ++ |
| 105 | ++++ | ++ |
| 106 | N/A | N/A |
| 107 | N/A | N/A |
| 108 | +++ | ++ |
| 109 | ++++ | ++ |
| 110 | ++ | +++ |
| 111 | N/A | N/A |
| Quickmist^{®} | +++++ | ++++ |

| | | |
|---|---|---|
| Evaluation: + = low irritation; +++++ = High irritation | | |

## Claims

1. A liquid mouth spray device containing a liquid mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10.

2. A liquid mouth spray device containing a liquid mucoadhesive oromucosal formulation according to any of the preceding claims, wherein the formulation also comprises nicotine that is not in complex with at least one mucoadhesive water-soluble anionic polymer.

3. A liquid mouth spray device containing a liquid mucoadhesive oromucosal formulation according to any of the preceding claims, wherein the at least one mucoadhesive water-soluble anionic polymer may be selected from a combination of one or more of xanthan gum(s), carrageenan(s) and carbomer(s).

4. A liquid mouth spray device containing a liquid mucoadhesive oromucosal formulation according to any of the preceding claims, wherein the concentration of the at least one mucoadhesive water-soluble anionic polymer in the formulation is between 0.1 w/w% to 2 w/w%, such as between 0.25 w/w% and 2 w/w%, such as between 0.25 w/w% and 1 w/w%, such as between 0.25 w/w% and 0,75 w/w% such as between 0.5 w/w% and 2 w/w%, such as between 0.5 w/w% and 1.5 w/w%, such as between 0.6 w/w% and 1 w/w%, such as between 0.6 w/w% and 0.8 w/w%.

5. A liquid mouth spray device containing a liquid mucoadhesive oromucosal formulation according to any of the preceding claims, wherein the formulation further comprises at least one preservative.

6. A liquid mouth spray device containing a liquid mucoadhesive oromucosal formulation according to claim 5, wherein the at least one preservative is selected from alcohols such as mono-alcohols such as ethanol, diols such as propylene glycol or polyalcohols or chlorhexidine.

7. A liquid mouth spray device containing a liquid mucoadhesive oromucosal formulation according to any of the preceding claims, wherein the pH of the formulation is between 8.5 and 10, such as between 8.5 and 9.5 or such as between 9 and 10.

8. A liquid mouth spray device containing a liquid mucoadhesive oromucosal formulation according to any of the preceding claims wherein the concentration of the nicotine in the formulation is between 0.1 w/w% and 10 w/w%, such as between 0.1 w/w% to 7 w/w%, such as between 0.25 w/w% and 5 w/w%, such as between 0.5 w/w% and 4 w/w%, such as between 0.5 w/w% and 3 w/w% such as between 0.5 w/w% and 2 w/w%, such as between 0.75 w/w% and 2 w/w%, such as between 1 w/w% and 2 w/w%, such as between 1.2 w/w% and 1.8 w/w%.

9. A Method of producing a liquid mouth spray device containing a liquid mucoadhesive oromucosal formulation comprising a nicotine complex and at least one preservative, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w, the method comprising the steps of:
- Mixing water with the at least one preservative
- Adding nicotine to the mixture
- Adding the at least one mucoadhesive water-soluble anionic polymer to the mixture
- Adjusting the pH of the formulation to between 8 and 10.

10. A Method of producing a liquid mouth spray device containing a liquid mucoadhesive oromucosal formulation comprising a nicotine complex and at least one preservative, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w, the method comprising the steps of:
- Mixing water with nicotine
- Adding the at least one mucoadhesive water-soluble anionic polymer to the mixture
- Adding the at least one preservative to the mixture
- Adjusting the pH of the formulation to between 8 and 10.

11. A method according to claim 9 or 10, wherein the pH of the formulation is adjusted to between 8.5 and 10, such as between 8.5 and 9.5 or such as between 9 and 10.

12. A liquid or gel mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10, for use as a medicament.

13. A liquid or gel mucoadhesive oromucosal formulation comprising a nicotine complex, said nicotine complex comprising nicotine in ionic complex with at least one mucoadhesive water-soluble anionic polymer, wherein the concentration of the at least one mucoadhesive water-soluble anionic polymer in the formulation is between 0.1 w/w% and 4.0 w/w%, and wherein the pH of the formulation is between 8 and 10, for use in obtaining nicotine relief by administration to the oral cavity.

## Patentansprüche

1. Vorrichtung für ein flüssiges Mundspray, die eine flüssige mukoadhäsive oromukosale Formulierung, umfassend einen Nikotinkomplex, enthält, der Nikotinkomplex umfassend Nikotin in ionischem Komplex mit mindestens einem mukoadhäsiven wasserlöslichen anionischen Polymer, wobei die Konzentration des mindestens einen mukoadhäsiven wasserlöslichen anionischen Polymers in der Formulierung zwischen 0,1 Gew.-% und 4,0 Gew.-% beträgt und wobei der pH-Wert der Formulierung zwischen 8 und 10 beträgt.

2. Vorrichtung für ein flüssiges Mundspray, die eine flüssige mukoadhäsive oromukosale Formulierung nach einem der vorstehenden Ansprüche enthält, wobei die Formulierung ebenso Nikotin, das nicht in dem Komplex mit mindestens einem mukoadhäsiven wasserlöslichen anionischen Polymer ist, umfasst.

3. Vorrichtung für ein flüssiges Mundspray, die eine flüssige mukoadhäsive oromukosale Formulierung nach einem der vorstehenden Ansprüche enthält, wobei das mindestens eine mukoadhäsive wasserlösliche anionische Polymer aus einer Kombination von einem oder mehreren von Xanthangummi(s), Carrageenan(en) und Carbomer(en) ausgewählt sein kann.

4. Vorrichtung für ein flüssiges Mundspray, die eine flüssige mukoadhäsive oromukosale Formulierung nach einem der vorstehenden Ansprüche enthält, wobei die Konzentration des mindestens einen mukoadhäsiven wasserlöslichen anionischen Polymers in der Formulierung zwischen 0,1 Gew.-% bis 2 Gew.-%, wie zwischen 0,25 Gew.-% und 2 Gew.-%, wie zwischen 0,25 Gew.-% und 1 Gew.-%, wie zwischen 0,25 Gew.-% und 0,75 Gew.-%, wie zwischen 0,5 Gew.-% und 2 Gew.-%, wie zwischen 0,5 Gew.-% und 1,5 Gew.-%, wie zwischen 0,6 Gew.-% und 1,0 Gew.-%, wie zwischen 0,6 Gew.-% und 0,8 Gew.-%, beträgt.

5. Vorrichtung für ein flüssiges Mundspray, die eine flüssige mukoadhäsive oromukosale Formulierung nach einem der vorstehenden Ansprüche enthält, wobei die Formulierung ferner mindestens ein Konservierungsmittel umfasst.

6. Vorrichtung für ein flüssiges Mundspray, die eine flüssige mukoadhäsive oromukosale Formulierung nach Anspruch 5 enthält, wobei das mindestens eine Konservierungsmittel aus Alkoholen wie Monoalkoholen wie Ethanol, Diolen wie Propylenglycol oder Polyalkoholen oder Chlorhexidin ausgewählt ist.

7. Vorrichtung für ein flüssiges Mundspray, die eine flüssige mukoadhäsive oromukosale Formulierung nach einem der vorstehenden Ansprüche enthält, wobei der pH-Wert der Formulierung zwischen 8,5 und 10, wie zwischen 8,5 und 9,5 oder wie zwischen 9 und 10, beträgt.

8. Vorrichtung für ein flüssiges Mundspray, die eine flüssige mukoadhäsive oromukosale Formulierung nach einem der vorstehenden Ansprüche enthält, wobei die Konzentration des Nikotins in der Formulierung zwischen 0,1 Gew.-% und 10 Gew.-%, wie zwischen 0,1 Gew.-% und 7 Gew.-%, wie zwischen 0,25 Gew.-% und 5 Gew.-%, wie zwischen 0,5 Gew.-% und 4 Gew.-%, wie zwischen 0,5 Gew.-% und 3 Gew.-%, wie zwischen 0,5 Gew.-% und 2 Gew.-%, wie zwischen 0,75 Gew.-% und 2 Gew.-%, wie zwischen 1 Gew.-% und 2 Gew.-%, wie zwischen 1,2 Gew.-% und 1,8 Gew.-%, beträgt.

9. Verfahren zum Herstellen einer Vorrichtung für ein flüssiges Mundspray, die eine flüssige mukoadhäsive oromukosale Formulierung, umfassend einen Nikotinkomplex und mindestens ein Konservierungsmittel, enthält, der Nikotinkomplex umfassend Nikotin in ionischem Komplex mit mindestens einem mukoadhäsiven wasserlöslichen anionischen Polymer, wobei die Konzentration des mindestens einen mukoadhäsiven wasserlöslichen anionischen Polymers in der Formulierung zwischen 0,1 Gew.-% und 4,0 Gew.-% beträgt, das Verfahren umfassend die Schritte:
- Mischen von Wasser mit dem mindestens einen Konservierungsmittel
- Hinzufügen von Nikotin zu der Mischung
- Hinzufügen des mindestens einen mukoadhäsiven wasserlöslichen anionischen Polymers zu der Mischung
- Einstellen des pH-Werts der Formulierung auf zwischen 8 und 10.

10. Verfahren zum Herstellen einer Vorrichtung für ein flüssiges Mundspray, die eine flüssige mukoadhäsive oromukosale Formulierung, umfassend einen Nikotinkomplex und mindestens ein Konservierungsmittel, enthält, der Nikotinkomplex umfassend Nikotin in ionischem Komplex mit mindestens einem mukoadhäsiven wasserlöslichen anionischen Polymer, wobei die Konzentration des mindestens einen mukoadhäsiven wasserlöslichen anionischen Polymers in der Formulierung zwischen 0,1 Gew.-% und 4,0 Gew.-% beträgt, das Verfahren umfassend die Schritte:
- Mischen von Wasser mit Nikotin
- Hinzufügen des mindestens einen mukoadhäsiven wasserlöslichen anionischen Polymers zu der Mischung
- Hinzufügen des mindestens einen Konservierungsmittels zu der Mischung
- Einstellen des pH-Werts der Formulierung auf zwischen 8 und 10.

11. Verfahren nach Anspruch 9 oder 10, wobei der pH-Wert der Formulierung auf zwischen 8,5 und 10, wie zwischen 8,5 und 9,5 oder wie zwischen 9 und 10 eingestellt wird.

12. Flüssige oder gelartige mukoadhäsive oromukosale Formulierung, umfassend einen Nikotinkomplex, der Nikotinkomplex umfassend Nikotin in ionischem Komplex mit mindestens einem mukoadhäsiven wasserlöslichen anionischen Polymer, wobei die Konzentration des mindestens einen mukoadhäsiven wasserlöslichen anionischen Polymers in der Formulierung zwischen 0,1 Gew.-% und 4,0 Gew.-% beträgt und wobei der pH-Wert der Formulierung zwischen 8 und 10 beträgt, zur Verwendung als ein Arzneimittel.

13. Flüssige oder gelartige mukoadhäsive oromukosale Formulierung, umfassend einen Nikotinkomplex, der Nikotinkomplex umfassend Nikotin in ionischem Komplex mit mindestens einem mukoadhäsiven wasserlöslichen anionischen Polymer, wobei die Konzentration des mindestens einen mukoadhäsiven wasserlöslichen anionischen Polymers in der Formulierung zwischen 0,1 Gew.-% und 4,0 Gew.-% beträgt und wobei der pH-Wert der Formulierung zwischen 8 und 10 beträgt, zur Verwendung in einem Erlangen einer Nikotinabhilfe durch Verabreichung in die Mundhöhle.

## Revendications

1. Dispositif de pulvérisation buccal liquide contenant une formulation oromuqueuse mucoadhésive liquide comprenant un complexe de nicotine, ledit complexe de nicotine comprenant de la nicotine en complexe ionique avec au moins un polymère anionique hydrosoluble mucoadhésif, dans lequel la concentration de l'au moins un polymère anionique hydrosoluble mucoadhésif dans la formulation est comprise entre 0,1 % en p/p et 4,0 % en p/p et dans lequel le pH de la formulation est compris entre 8 et 10.

2. Dispositif de pulvérisation buccal liquide contenant une formulation oromuqueuse mucoadhésive liquide selon l'une quelconque des revendications précédentes, dans lequel la formulation comprend également de la nicotine qui n'est pas en complexe avec au moins un polymère anionique hydrosoluble mucoadhésif.

3. Dispositif de pulvérisation buccal liquide contenant une formulation oromuqueuse mucoadhésive liquide selon l'une quelconque des revendications précédentes, dans laquelle l'au moins un polymère anionique hydrosoluble mucoadhésif peut être choisi parmi une combinaison d'une ou plusieurs gommes xanthanes, d'un ou plusieurs carraghénanes et d'un ou plusieurs carbomères.

4. Dispositif de pulvérisation buccal liquide contenant une formulation oromuqueuse mucoadhésive liquide selon l'une quelconque des revendications précédentes, dans lequel la concentration de l'au moins un polymère anionique hydrosoluble mucoadhésif dans la formulation est comprise entre 0,1 % en p/p et 2 % en p/p, tel qu'entre 0,25 % en p/p et 2 % en p/p, tel qu'entre 0,25 % en p/p et 1 % en p/p, tel qu'entre 0,25 % en p/p et 0,75 % en p/p tel qu'entre 0,5 % en p/p et 2 % p/p, tel qu'entre 0,5 % en p/p et 1,5 % en p/p, tel qu'entre 0,6 % en p/p et 1 % en p/p, tel qu'entre 0,6 % en p/p et 0,8 % en p/p.

5. Dispositif de pulvérisation buccal liquide contenant une formulation oromuqueuse mucoadhésive liquide selon l'une quelconque des revendications précédentes, dans lequel la formulation comprend en outre au moins un conservateur.

6. Dispositif de pulvérisation buccal liquide contenant une formulation oromuqueuse mucoadhésive liquide selon la revendication 5, dans lequel l'au moins un conservateur est choisi parmi des alcools tels que des mono-alcools tels que l'éthanol, des diols tels que le propylène glycol ou les polyalcools ou la chlorhexidine.

7. Dispositif de pulvérisation buccal liquide contenant une formulation oromuqueuse mucoadhésive liquide selon l'une quelconque des revendications précédentes, dans lequel le pH de la formulation est compris entre 8,5 et 10, tel qu'entre 8,5 et 9,5 ou tel qu'entre 9 et 10.

8. Dispositif de pulvérisation buccal liquide contenant une formulation oromuqueuse mucoadhésive liquide selon l'une quelconque des revendications précédentes, dans laquelle la concentration de la nicotine dans la formulation est comprise entre 0,1 % en p/p et 10 % en p/p, tel qu'entre 0,1 % en p/p et 7 % en p/p, tel qu'entre 0,25 % en p/p et 5 % en p/p, tel qu'entre 0,5 % en p/p et 4 % en p/p, tel qu'entre 0,5 % en p/p et 3 % en p/p, tel qu'entre 0,5 % en p/p et 2 % en p/p, tel qu'entre 0,75 % en p/p et 2 % en p/p, tel qu'entre 1 % en p/p et 2 % en p/p, tel qu'entre 1,2 % en p/p et 1,8 % en p/p.

9. Procédé de production d'un dispositif de pulvérisation buccal liquide contenant une formulation oromuqueuse mucoadhésive liquide comprenant un complexe de nicotine et au moins un conservateur, ledit complexe de nicotine comprenant de la nicotine en complexe ionique avec au moins un polymère anionique hydrosoluble mucoadhésif, dans lequel la concentration de l'au moins un polymère anionique hydrosoluble mucoadhésif dans la formulation est compris entre 0,1 % en p/p et 4,0 % en p/p, le procédé comprenant les étapes consistant à :
- Mélanger de l'eau avec l'au moins un conservateur
- Ajouter de la nicotine au mélange
- Ajouter l'au moins un polymère anionique hydrosoluble mucoadhésif au mélange
- Régler le pH de la formulation entre 8 et 10.

10. Procédé de production d'un dispositif de pulvérisation buccal liquide contenant une formulation oromuqueuse mucoadhésive liquide comprenant un complexe de nicotine et au moins un conservateur, ledit complexe de nicotine comprenant de la nicotine en complexe ionique avec au moins un polymère anionique hydrosoluble mucoadhésif, dans lequel la concentration de l'au moins un polymère anionique hydrosoluble mucoadhésif dans la formulation est comprise entre 0,1 % en p/p et 4,0 % en p/p, le procédé comprenant les étapes consistant à :
- Mélanger de l'eau avec de la nicotine
- Ajouter l'au moins un polymère anionique hydrosoluble mucoadhésif au mélange
- Ajouter l'au moins un conservateur au mélange
- Régler le pH de la formulation entre 8 et 10.

11. Procédé selon la revendication 9 ou 10, dans lequel le pH de la formulation est réglé entre 8,5 et 10, tel qu'entre 8,5 et 9,5 ou tel qu'entre 9 et 10.

12. Formulation oromuqueuse mucoadhésive liquide ou en gel comprenant un complexe de nicotine, ledit complexe de nicotine comprenant de la nicotine en complexe ionique avec au moins un polymère anionique hydrosoluble mucoadhésif, dans laquelle la concentration de l'au moins un polymère anionique hydrosoluble mucoadhésif dans la formulation est comprise entre 0,1 % en p/p et 4,0 % en p/p, et dans laquelle le pH de la formulation est compris entre 8 et 10, destinée à être utilisée en tant que médicament.

13. Formulation oromuqueuse mucoadhésive liquide ou en gel comprenant un complexe de nicotine, ledit complexe de nicotine comprenant de la nicotine en complexe ionique avec au moins un polymère anionique hydrosoluble mucoadhésif, dans laquelle la concentration de l'au moins un polymère anionique hydrosoluble mucoadhésif dans la formulation est comprise entre 0,1 % en p/p et 4,0 % en p/p, et dans laquelle le pH de la formulation est compris entre 8 et 10, destinée à être utilisée dans l'obtention d'un soulagement de nicotine par administration à la cavité buccale.
